# EUROPEAN PATENT SPECIFICATION

(11) **EP 3 652 748 B1**
(45) Date of publication and mention of the grant of the patent: **16.10.2024**
(21) Application number: 18745503.5
(22) Date of filing: 05.07.2018
(51) Int. Cl.: G16H 40/40

(54) **PREDICTIVE MAINTENANCE FOR LARGE MEDICAL IMAGING SYSTEMS**
PRÄDIKTIVE WARTUNG FÜR GROSSE MEDIZINISCHE BILDGEBUNGSSYSTEME
MAINTENANCE PRÉDICTIVE POUR SYSTÈMES D'IMAGERIE MÉDICALE DE GRANDE TAILLE

(30) Priority: 10.07.2017 US 201762530385 P
(43) Date of publication of application: 20.05.2020
(73) Proprietor: Koninklijke Philips N.V., 5656 AG Eindhoven (NL)
(72) Inventor: MAVRIEUDUS, Dimitros, 5656 AE Eindhoven (NL); BOUMANS, Michael, Leonardus, Helena, 5656 AE Eindhoven (NL)
(74) Representative: Philips Intellectual Property & Standards
(86) International application number: PCT/EP2018/068180
(87) International publication number: WO 2019/011765

(56) References cited:
- US-A1- 2015 227 838
- ROSHAN ALWIS ET AL: "Machine Learning Techniques for Predictive Maintenance", INFOQ, 21 May 2017 (2017-05-21), XP055511190, Retrieved from the Internet <URL:https://www.infoq.com/articles/machine-learning-techniques-predictive-maintenance> [retrieved on 20181001]
- XIAO-SHENG SI ET AL: "Remaining useful life estimation A review on the statistical data driven approaches", EUROPEAN JOURNAL OF OPERATIONAL RESEARCH, ELSEVIER, AMSTERDAM, NL, vol. 213, no. 1, 17 November 2010 (2010-11-17), pages 1 - 14, XP028205970, ISSN: 0377-2217, [retrieved on 20101124], DOI: 10.1016/J.EJOR.2010.11.018

## Description

### FIELD

The following relates generally to the medical imaging system (including image guided therapy, iGT) maintenance arts, medical imaging system failure prediction arts, and related arts, in particular a predictive maintenance alerting method, a non-transitory storage medium storing instructions readable and executable by an electronic processor to perform such method and a predictive maintenance alerting device for performing such method.

### BACKGROUND

Medical imaging devices include very complex systems such as magnetic resonance imaging (MRI) devices, ultrasound imaging devices, digital radiography (DR) devices, transmission computed tomography (CT) imaging devices, emission imaging systems such as positron emission tomography (PET) imaging devices and gamma cameras for single photon emission computed tomography (SPECT) imaging, hybrid systems that provide multiple modalities in a single device, e.g. a PET/CT or SPECT/CT imaging device, and imaging devices designed for guiding biopsies or other interventional medical procedures, commonly referred to as image guided therapy (iGT) devices. These are merely illustrative examples.

Modern medical imaging devices present unusual challenges from a maintenance standpoint. These imaging devices can be very complex, e.g. in some medical imaging systems there may be on the order of 19,000 serviced components. These devices are also being used for increasingly diverse types of medical imaging and procedures: for example, whereas traditionally iGT machines have been used for a limited number of procedures (such as diagnostic treatments to prepare for open heart surgery), iGT machines are now used for a much wider array of minimal invasive treatments (e.g. heart-valve replacement). Machine complexity can also be understood in terms of data production: for example, a medical imaging devices installation base may generate 10 TB/year of log data, i.e. 2-4 GB/day (mostly in the form of compressed text files).

A further difficulty in diagnosis of problems with a medical imaging device is that some data resources may have data quality issues. For example, uncertainty or quality of the contents of service call data can be related to the human-decision factors (where decisions are made on-the-spot, based on factors that cannot always be quantified, such as customer relations). Moreover, certain machine problems may be addressed in multiple ways (calibration versus component replacement) leading to ambiguities when using past service histories to predict future problem solutions.

US patent application publication 20150227838A1 discloses a computer-implemented method of building a model for predicting failure of a machine.

The following discloses a new and improved systems and methods.

### SUMMARY

In one disclosed aspect, a non-transitory storage medium stores instructions readable and executable by an electronic processor to perform a predictive maintenance alerting method comprising: receiving time stamped machine log data and time stamped service log data for a medical imaging device via an electronic network; deriving features from the received time stamped machine log data and time stamped service log data, including deriving at least one of (i) built failure mode features each comprising a combination of two or more features that together represent a failure mode of a component and (ii) built failure resolution features each comprising a combination of two or more features that together represent a failure of a component and a resolution of the failure of the component; applying a set of models of component groups to the derived features to generate maintenance alerts wherein each component of the medical imaging device is exclusively a member of a single component group; and transmitting the generated maintenance alerts to a service center via the electronic network.

In another disclosed aspect, a predictive maintenance alerting device comprises a server computer operatively connected with an electronic network to receive time stamped machine log data and time stamped service log data from a medical imaging device via the electronic network and to transmit maintenance alerts to a service center via the electronic network. A non-transitory storage medium stores instructions readable and executable by the server computer to perform a predictive maintenance alerting method including deriving features from the received time stamped machine log data and time stamped service log data, including deriving at least one of (i) built failure mode features each comprising a combination of two or more features that together represent a failure mode of a component and (ii) built failure resolution features each comprising a combination of two or more features that together represent a failure of a component and a resolution of the failure of the component, and applying a set of models of component groups to the derived features to generate the maintenance alerts. Each model of the set of models of component groups comprises a heterogeneous model including a machine learned analytical model representing the component group with embedded statistical remaining useful lifetime models of the components of the component group.

In another disclosed aspect, a predictive maintenance alerting method comprises: receiving time stamped machine log data and time stamped service log data for a medical imaging device at a server computer via an electronic network; deriving features from the received time stamped machine log data and time stamped service log data, including deriving at least one of (i) built failure mode features each comprising a combination of two or more features that together represent a failure mode of a component and (ii) built failure resolution features each comprising a combination of two or more features that together represent a failure of a component and a resolution of the failure of the component; applying a set of models of component groups to the derived features to generate maintenance alerts; and transmitting the generated maintenance alerts from the server computer to a service center via the electronic network. The deriving and applying are suitably performed by the electronic server.

One advantage resides in providing maintenance alerting for complex medical imaging systems commonly including ten thousand or more components.

Another advantage resides in providing such maintenance alerting while reducing component-level and component group-level ambiguities.

Another advantage resides in providing such maintenance alerting while reducing component-level and component group-level dependencies.

Another advantage resides in providing such maintenance alerting which integrates machine learned analytical modeling at the component group level with statistical remaining useful lifetime modeling of the components.

Another advantage resides in providing such maintenance alerting employing built features to efficiently capture and process failure modes.

Another advantage resides in providing such maintenance alerting employing built features to efficiently capture and process different failure resolution solutions.

Another advantage resides in providing such maintenance alerting which maximizes correct maintenance alerts while limiting erroneous maintenance alerts so as to provide effective alerting without overburdening service personnel.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The invention may take form in various components and arrangements of components, and in various steps and arrangements of steps. The drawings are only for purposes of illustrating the preferred embodiments and are not to be construed as limiting the invention. In drawings presenting log or service call data, certain identifying information has been redacted by use of superimposed redaction boxes.
FIGURE 1 diagrammatically illustrates a predictive maintenance alerting device and its surrounding environment including a medical imaging device for which maintenance alerts are generated, along with a service center to which the maintenance alerts are communicated.
FIGURE 2 shows an illustrative fragment of machine log data.
FIGURE 3 shows illustrative service log data for a service call.
FIGURE 4 shows illustrative service log data for another service call.
FIGURE 5 diagrammatically illustrates a process for training the component group models of the predictive maintenance alerting device of claim 1.
FIGURE 6 shows an illustrative display of maintenance alerts generated by the predictive maintenance alerting device of FIGURE 1 and suitably shown on a display at the service center.

### DETAILED DESCRIPTION

In maintenance services, three approaches are typical: reactive maintenance, proactive maintenance, and predictive maintenance. The difference between these three approaches relates to who initiates the call. In reactive maintenance, the customer initiates the call; whereas, in proactive or predictive maintenance, the service provider initiates the call. Predictive and proactive maintenance relies on statistical, machine learning, data mining and/or optimization models that are developed using historical data relevant to the device being maintained. For medical imaging devices, these data usually belong in the following categories: service call logs (contain information about when a customer called to report a maintenance-related problem and which actions were taken to address the issue); and machine log data (which herein is broadly understood to encompass usage data, i.e. information about how frequently each component of the system was used, and sensor data, i.e. information extracted from sensors, such as temperature or so forth).

Predictive maintenance for medical imaging devices is particularly challenging due to the complexity of the devices, and the large amounts of log data generated. To illustrate, some image-guided therapy (iGT) systems manufactured by Koninklijke Philips N.V. include around 19,000 different components to be maintained. In terms of data logging, an installed base of medical imaging devices may generate 10 TB of data per year, or 2-4 GB per day. These data volumes are for the compressed data, mostly in the form of compressed text files.

In predictive maintenance device and method embodiments disclosed herein, these difficulties are effectively addressed. The complexity of hardware is addressed by identifying correlated groups of components (out of the typically at least 10,000 components making up the medical imaging device) that can be optimally modelled together. Service call ambiguity is reduced as calls that have common root causes can be identified, which is used to strengthen the robustness of the predicted maintenance alerts (for example to achieve "first-time-right" response to customer call). The handling of large and heterogeneous features may be addressed by identifying relevant features and data resources that are relevant to the component group at hand. The handling of "context" around machine log messages is accounted for in part by the use of built features that combine features (e.g. log messages) that together represent a failure mode, or a failure resolution. This facilitates disambiguation of certain errors in the machine logs that may take different interpretations depending on their surrounding errors or messages. In some embodiments, heterogeneous predictive maintenance models are employed, by which statistics are used to sharpen the predictive alert.

To address the challenges associated with providing useful maintenance alerts for complex medical imaging device, disclosed predictive maintenance alerting devices and methods receive input including Service calls logs and machine log data (including usage data and sensor data). Groups of components (out of the, e.g., ~19000 contained in the iGT systems) are identified that are optimally grouped together in the predictive models (i.e. they are related to common root causes). In the training phase, relevant service calls for component groups are found, and suitable features are selected and/or constructed (e.g., built features that capture both the failure and its resolution, or that capture the particular mode of failure of a component). The component group models are then optimized.

To provide an illustrative example, consider a component group for a footswitch of an iGT device. The footswitch is a component that controls fluoroscopy and exposure while iGT systems are used. The group of components may include the footswitch, the cable connected to the footswitch, the components that enable X-rays in response to operation of the footswitch, and perhaps other related components. To build a model for this "footswitch" component group, the components of the group are first selected. This includes hardware, e.g. selected component ids that cover 15years of hardware development, and software, e.g. selected units that cover 15 years of software development. Next, it is determined in service call data the calls relevant to the footswitch component group (which, again, may contain certain components attached to the footswitch such as the attached cable which can generate similar error messages in the machine log files when broken). Built features are constructed that identify, for example, cases where the footswitch is used but X-ray is not enabled (i.e. associating the footswitch component with a particular failure mode). Frequency and repetition of this built feature are optimized for model performance. The model is then optimized for performance according to business needs and domain expertise.

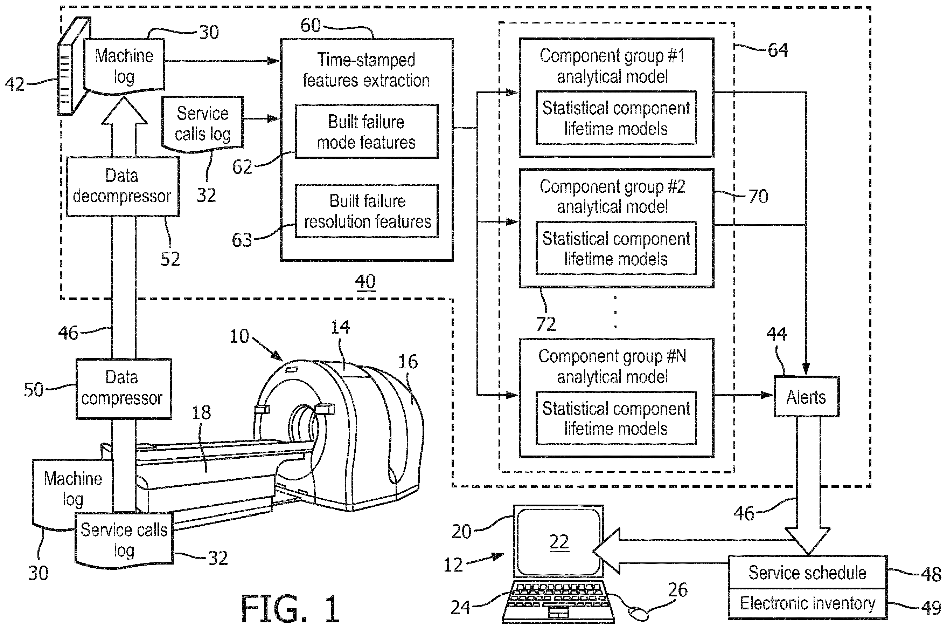

With reference to FIGURE 1, an illustrative predictive maintenance alerting device is diagrammatically shown, along with its relevant surrounding environment including a medical imaging device **10** for which maintenance alerts are generated, along with a service center **12** to which the maintenance alerts are communicated. By way of non-limiting example, the illustrative medical imaging device **10** is a hybrid imaging system including a first-modality **14** and a second-modality **16** serviced by a common patient couch or transport device **18**. For example, the first modality **14** may be transmission computed tomography (CT) imaging, and the second modality **16** may be positron emission tomography (PET) imaging (thus, the medical imaging device **10** would be a PET/CT imaging device). This is merely an example, and more generally the medical imaging device that is monitored to issue predictive maintenance alerts may be (by way of further non-limiting illustration) be a magnetic resonance imaging (MRI) device, a CT imaging device, an emission imaging device such as a PET imaging device or a gamma camera for single photon emission computed tomography (SPECT) imaging, a hybrid imaging device such as PET/CT or SPECT/CT, or an imaging device designed for guiding biopsies or other interventional medical procedures, commonly referred to as an image guided therapy (iGT) device.

The service center **12** is represented in diagrammatic FIGURE 1 by a service center workstation or service center computer **20** including a display **22** and one or more user input devices (e.g. an illustrative keyboard **24** and mouse **26**). The service center workstation or computer **20** may perform various service support functions, such as mapping locations of medical institutions and individual medical image devices contracted for service by the service center **12**, coordinating, tracking, and logging service calls performed by human service agents, queuing or docketing service requests received from customers, and/or so forth. Of relevance for the predictive maintenance alerting devices disclosed herein, the service center workstation or computer **20** is programmed to receive and display maintenance alerts generated by the predictive maintenance alerting device. Service center personnel can then decide whether to follow up on any particular maintenance alert received from the predictive maintenance alerting device.

With continuing reference to FIGURE 1, the medical imaging device **10**, or systems or devices associated with the medical imaging device **10**, generate a machine log **30** (which, again, as used herein may include medical imaging device usage log data and/or sensor data acquired by sensors of the medical imaging device). Similarly, a service log **32** is maintained, which records information about service calls, i.e. maintenance performed on the medical imaging device **10**.

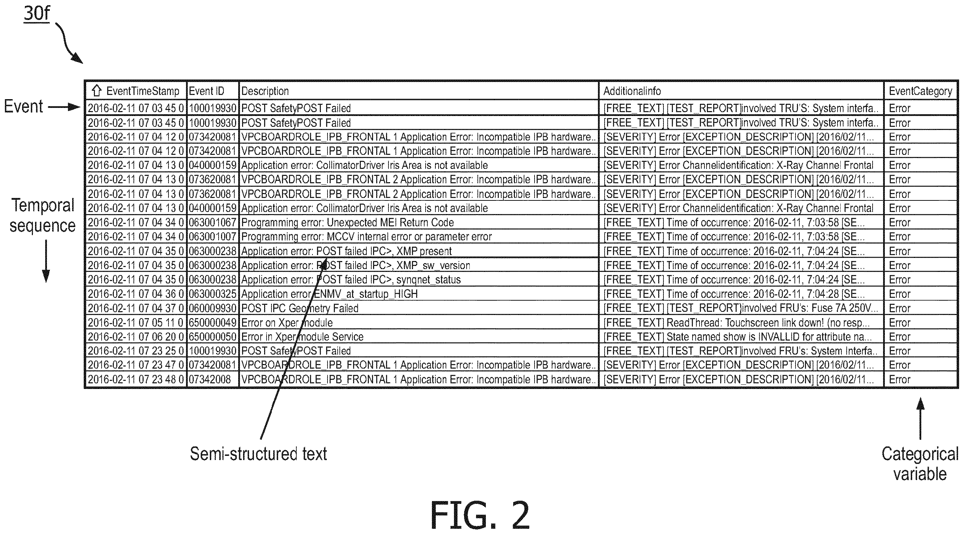

With brief reference to FIGURE 2, an illustrative fragment **30f** of a possible formulation of the machine log **30** is shown. In this illustrative example, each event (or message) occupies a single row of the machine log data. The leftmost column labeled "EventTimeStamp" stores timestamps of logged events or messages. An "eventID" column stores a unique identifier for each machine log entry. A "Description" column stores a semi-structured text description of the event or message. An "AdditionalInfo" column stores further information, again in a semi-structured textual format. An "EventCategory" column stores a classification of each event or message of the machine log. The example of FIGURE 2 is merely a non-limiting illustrative example, and the machine log data can assume various other formulations, e.g. including additional, fewer, and/or different columns, different syntax, and/or so forth. In general, each entry of the machine log **30** is timestamped and includes a text-based description of the event or message, which may contain information such as sensor data, detected component faults, entries logging the start of an imaging procedure and subsequent entries logging operations performed during the imaging procedure, and/or so forth.

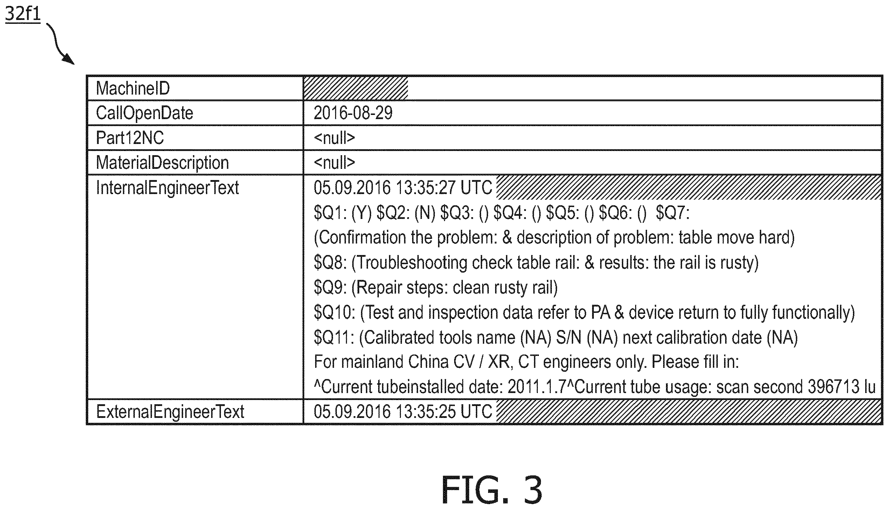

With reference to FIGURE 3, an illustrative entry **32f1** of a possible formulation of the service log **32** is shown, for a particular illustrative service call. As can be seen, each service call is again timestamped (here using the nomenclature "CallOpenDate"; a "CallCloseDate" is also contemplated) and stores a semi-structured text description of the service call including information collected and remedial action taken to resolve the problem.

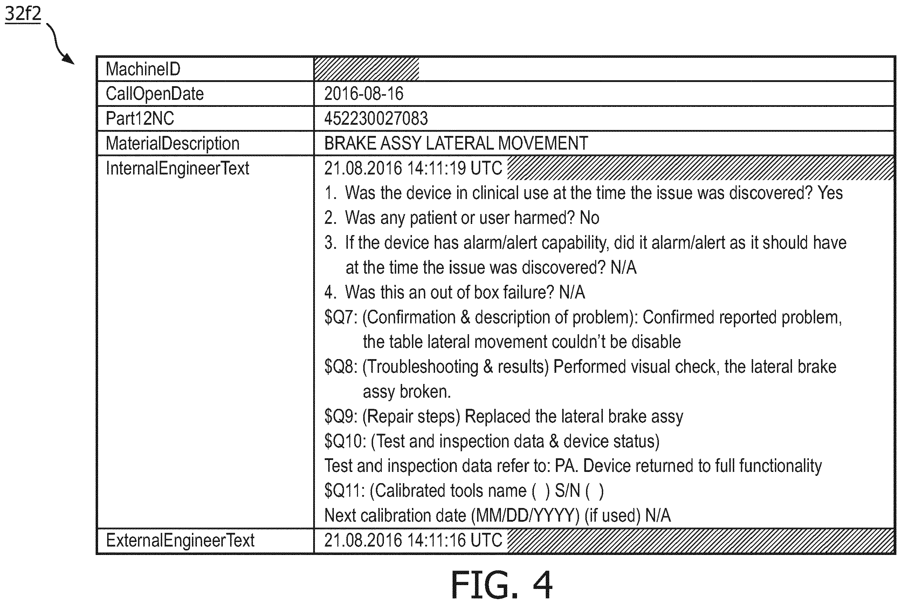

With reference to FIGURE 4, another illustrative entry **32f2** of the possible formulation of the service log **32** is shown, for another illustrative service call. Again, the service call entry is timestamped via the "CallOpenDate" and a semi-structured text description of the service call is stored. The examples of FIGURES 3 and 4 are merely non-limiting illustrative examples, and the service log data can assume various other formulations, e.g. including additional, less, and/or different information in different formats and/or using different syntax. In general, each entry of the service log **32** is timestamped and includes a text-based description of the service call including any information collected and remedial actions taken to resolve the problem. In some instances, a service call entry may not include resolution of the problem if the service call was indeed unable to resolve the problem. Moreover, a service call entry may include extraneous information - for example, a service person may perform routine maintenance operations unrelated to the component failure that was the genesis of the service call. There may also be "noise" in the sense that in some instances the remedial action taken may be "incorrect", i.e. may not actually solve the root cause of the component failure that was the basis of the service call.

With returning reference to FIGURE 1, a predictive maintenance alerting device **40** comprises a server computer **42** executing instructions stored on a non-transitory storage medium (not shown) and readable and executable by the server computer **42** to perform a predictive maintenance alerting method as disclosed herein. The server computer **42** may be variously embodied, e.g. as a single server computer, or as two or more server computers operatively connected to perform concurrent processing to implement the predictive maintenance alerting method, or as a combination of two or more computers operatively connected in an ad hoc fashion to perform the predictive maintenance alerting method (e.g. a cloud computing resource). While a server computer (or group or ad hoc combination of server computers) is generally preferred as such provides substantial computing capacity, in other embodiments it is contemplated for the disclosed predictive maintenance alerting method to be implemented on a desktop computer or other electronic processor if such has sufficient computing capacity. The non-transitory storage medium may, for example, be a hard disk drive, RAID, or other magnetic storage medium; a flash memory, solid state drive (SSD), or other electronic storage medium; an optical disk or other optical storage medium; various combinations thereof; or so forth.

The predictive maintenance alerting device **40** processes the machine log **30** and service log **32** of the medical imaging device **10** generate maintenance alerts **44** that are transmitted to the service center **12** (e.g., more particularly transmitted to the service center workstation or computer **20** in the illustrative embodiment). To this end, the electronic processor (illustrative server computer) **42** is operatively connected with an electronic network **46** to receive the time stamped machine log data **30** and time stamped service log data **32** via the electronic network **46** and to transmit the maintenance alerts **44** to the service center **12** via the electronic network **46**. The electronic network **46** is diagrammatically indicated in FIGURE 1 by data flow block arrows, and in physical implementations can be variously embodied as a hospital data network, the Internet, data networking infrastructure provided by a commercial Internet Service Provider (ISP), and/or so forth. The data flows between the medical imaging device **10** and the predictive maintenance alerting device **40** on the one hand, and between the predictive maintenance alerting device **40** and the service center **12** on the other hand, may employ different data networking hardware. For example, the former may include a hospital data network connected with the Internet connected with a local area network of which the server computer **42** is a part, while the latter may (again, by way of non-limiting illustrative example) include only the local area network hosting the server computer **42** if the server computer is located at the service center **12**, or may further include a commercial ISP if located elsewhere. These are merely a few non-limiting illustrative examples of possible implementations of the electronic network **46**.

As further diagrammatically indicated in FIGURE 1, the service center **12** or a server computer or other computing resource may optionally perform processing of the maintenance alerts **44**. For example, an electronic service schedule **48** may be implemented on a server or other computer to automatically or semi-automatically schedule a service call based on the maintenance alert **44**. For example, a proposed service call may be added to the service schedule **48**, which must be accepted by the customer via a web-based customer interface to the service schedule **48** or the like. The customer may have the option of re-scheduling or canceling the proposed service call, subject to availability of service personnel. Additionally or alternatively, an electronic inventory **49** may be implemented on such a server or other computer to automatically or semi-automatically order parts required (or likely to be required) to address the maintenance alert **44**, and/or to re-stock such parts in anticipation of a service call to resolve the maintenance alert. The electronic inventory **49** may also ship a part from physical inventory to the customer site preparatory to the anticipated service call. Approval by service personnel of parts ordering or shipping may be required, and the customer is preferably not billed for parts unless and until the parts are installed in the customer's device.

In a typical implementation, the logs **30**, **32** are uploaded to the predictive maintenance alerting device **40** on a daily basis, e.g. each morning as the machine **10** is brought online to provide imaging services. The logs **30**, **32** are illustrated in FIGURE 1 as associated with the medical imaging device **10**; however, the logs may be stored at any network-accessible server, RAID array, cloud storage locker, or the like. In some embodiments the logs may be maintained on a server or other network-based resource maintained by the manufacturer of the medical imaging device or by the service provider. To conserve bandwidth and speed up the data transfer, a data compressor **50** executing on a computer (not shown, disposed at or with or operatively connected with the resource that stores the logs) compresses the data logs **30**, **32** using any suitable compression algorithm (e.g. zip compression or variants thereof, tar compression or variants thereof, or so forth). Even with this compression, the compressed data logs **30**, **32** for a large medical imaging device may constitute a large quantity of data. A the predictive maintenance alerting device **40**, the server computer **42** executes a data decompressor **44** to decompress the compressed log data received at the predictive maintenance alerting device **40**, so as to reproduce (a copy of) the time stamped machine log data **30** and time stamped service log data **32** at the predictive maintenance alerting device **40**. While data compression is preferred to reduce bandwidth, increase data transfer speed, and reduce loading on the electronic network **46**, it is contemplated to omit the data compression components **50**, **52** and instead transmit the log data **30**, **32** in uncompressed form.

The predictive maintenance alerting device **40** generates the maintenance alerts **44** for the medical imaging device **10** by performing time-stamped features extraction **60** (optionally including deriving built features **62**, **63** as disclosed herein) from the received time stamped machine log data **30** and time stamped service log data **32**, and applying a set of models **64** of component groups to the derived features to generate the maintenance alerts **44**. By way of non-limiting illustration, in FIGURE 1 the set of models **64** of component groups is depicted as containing N models for N component groups.

The features extraction **60** is suitably performed by operations including parsing of the semi-structured text of the descriptions (and optional additional information) in the machine log **30** (e.g. see FIGURE 2) and service log **32** (see FIGURES 3 and 4). The choice of features in general depends on the nomenclature and syntax employed by the machine and service logging software. Some features may be numerical, e.g. a machine log entry reporting a temperature sensor reading may be formulated as a (sensor identifier, <temp value>) pair where <temp value> indicates the temperature read by the sensor. Some features may be binary, or may assume values chosen from a discrete set of possible values: for example, in the service log fragment **32f2** of FIGURE 4, a possible feature may be (table lateral brake assembly status = nonfunctional) where for this type of feature more generally the two possible values are "functional" or "nonfunctional". The parsing of the semi-structured text includes identifying component names or identifiers and associating log entries containing those with the named or identified component. Extracted features may also be classified by event category or other information. The timestamp of each entry that is converted to a feature is preferably retained as the timestamp of that feature.

The built features **62**, **63** are constructed as combinations of two or more constituent features, where each constituent feature is extracted from a log entry. The combinations are chosen to be more informative than the individual features extracted from individual log entries. Viewed another way, the built features provide additional context that is informative for predicting incipient maintenance issues.

By way of illustration, one type of built feature is a built failure mode feature **62**, suitably comprising a combination of two or more features that together represent a failure mode of a component. For example, considering the illustrative footswitch component mentioned previously, a built failure mode feature **62** may be constructed as the combination of a log entry indicating activation of the footswitch and a subsequent entry indicating the X-ray is not enabled. This built feature captures a failure mode of the footswitch in which activating the footswitch fails to perform the expected function of enabling X-rays. In constructing this built feature, the timestamps are preferably taken into account, i.e. the built feature preferably requires that the log entry indicating X-rays not enabled is time stamped subsequent to the log entry indicating the footswitch is activated, and moreover may require that there not be any intervening timestamped entry indicating an operation that would disable X-rays.

As another illustrative example, another type of built feature is a built failure resolution feature **63**, suitably comprising a combination of two or more features that together represent a failure of a component and a resolution of the failure of the component. For example, from the service log fragment **32f1** of FIGURE 3 a built failure resolution feature **63** may be constructed consisting of the combination of the feature "table movement hard" (or, in other embodiments, some more general term such as "problems with table movement") indicating the failure and "clean rail" indicating the resolution. From the service log fragment **32f2** of FIGURE 4, a built failure resolution feature **63** may be constructed consisting of combination of the feature (table lateral brake assembly status = nonfunctional) and the feature "lateral brake assembly replaced".

The set of models **64** of component groups has certain features to facilitate maintenance prediction for large medical imaging devices. The assignment of components to component groups is preferably done to group together components related to a common root cause. In some embodiments, each component of the medical imaging device is exclusively a member of a single component group, and hence is modeled by a single model of the set of models **64**. This approach reduces ambiguity that could result if, for example, two different models generated maintenance alerts for the same component.

In other contemplated embodiments, some components may be assigned to two or more different groups, and hence be modeled by different models. For instance, considering the example of the footswitch operable to enable X-ray, a component that receives input from the footswitch and generates a signal that operates to enable X-ray could be usefully included in both a footswitch component group and an X-ray component group. In such cases, the component's membership in multiple groups is preferably kept low, e.g. preferably no more than two or three groups.

As diagrammatically shown in FIGURE 1, in some embodiments at least one model of the set of models **64** of component groups (and preferably all models) comprises a heterogeneous model including a machine-learned analytical model **70** representing the component group, with embedded statistical remaining useful lifetime models **72** of the components of the component group. This type of heterogeneous model advantageously leverages machine learning to provide the analytical model **70** to empirically capture the complex interrelations between components of the component group, while also using the remaining useful lifetime models **72** to capture statistical failure likelihoods and to assign time horizons for when a problematic component is likely to fail.

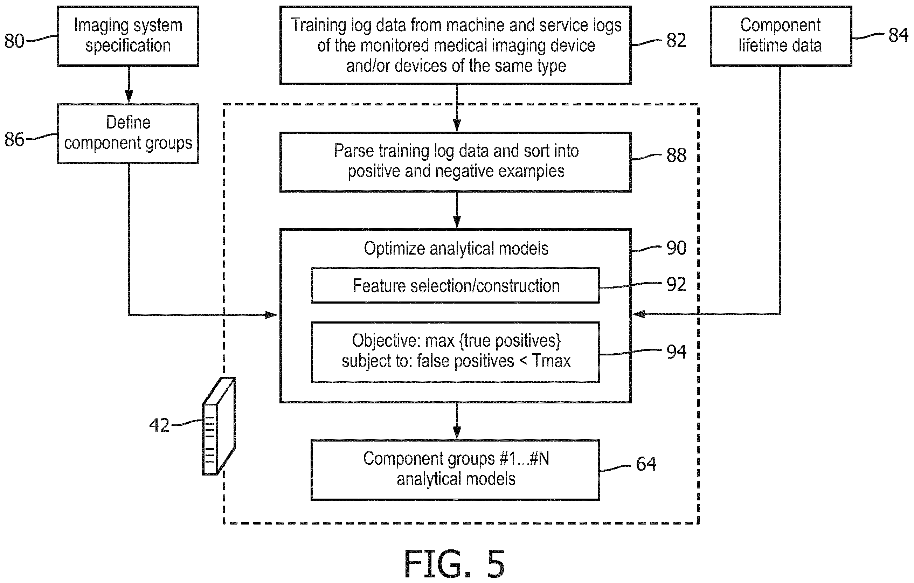

With reference to FIGURE 5, one suitable method for constructing the set of models **64** is described. The illustrative method is implemented by the server computer **42** executing instructions read from a non-transitory storage medium, but could instead be implemented on a different computer. The inputs include the imaging system specification **80** (e.g. identification of the components of the component group whose model is being trained along with other salient parameters of the medical imaging device), training log data **82**, and component lifetime data **84**. The training log data **82** comprises timestamped machine log data and timestamped service log data, and may be obtained from the medical imaging device **10** for which the set of models is intended, and/or may be obtained from one or more other medical imaging devices of a same type.

In an operation **86**, the component groups are defined. This is typically a manual operation, performed by system engineers having expert knowledge as to which components functionally cooperate as sub-systems of the medical imaging device. An operation **88** parses the training log data **82**. This parsing may be similar to that of the already-described time-stamped features extraction **60**, but optionally may extract more features than those of the extraction **60** with the intent of performing statistically based feature selection, e.g. based on discriminativeness of the features. The operation **88** further extracts positive training data and negative training data from the parsed training log data. The positive training data comprise training data having time stamps pre-dating a failure of a component of component group, while the negative training data comprise training data having time stamps post-dating a failure of a component of component group or training data from a medical imaging device that has never had a failure of the component of component group.

In an operation **90**, machine learning is applied to train the analytical model using the positive training data and the negative training data. The operation **88** optionally includes a feature selection phase **92** that, for a given model, selects a sub-set of the features extracted in the operation **88** on the basis of relevance or discriminativeness. For example, relevance may be determined based on whether the feature is associated with any of the components of the component group, while discriminativeness may be assessed based on whether the feature occurs more frequently in the positive training data versus the negative training data, or vice versa. (By contrast, if a feature occurs at similar frequencies in both the positive training data and the negative training data, then its discriminativeness is likely to be low and may be discarded during the feature selection).

The component lifetime data **84** is used to construct the statistical remaining useful lifetime models **72** of the components of the component group. These component-level statistical remaining useful lifetime models **72** are embedded in the analytical model **70** of the component group, which is then trained by machine learning to optimize the model with respect to an objective **94**. Various objective formulations may be employed; however, in one preferred embodiment, the objective comprises maximizing true positive maintenance alerts for the component group (where "true positive" means the model correctly predicts a maintenance operation that actually occurs in the positive training data) subject to an upper limit on false positive maintenance alerts for the component group (where "false positive" means the model incorrectly predicts a maintenance alert that does not show up in the negative training data). This choice of objective **94** advantageously maximizes the rate of "correct" maintenance alerts (as measured by the true positives rate) which are useful for driving maintenance activity, while avoiding issuing more than a threshold number of false positive maintenance alerts which could otherwise overwhelm the resources of the service center **12**. The trained models then form the set of models **64** of component groups which are applied by the predictive maintenance alerting device **40** previously described with reference to FIGURE 1.

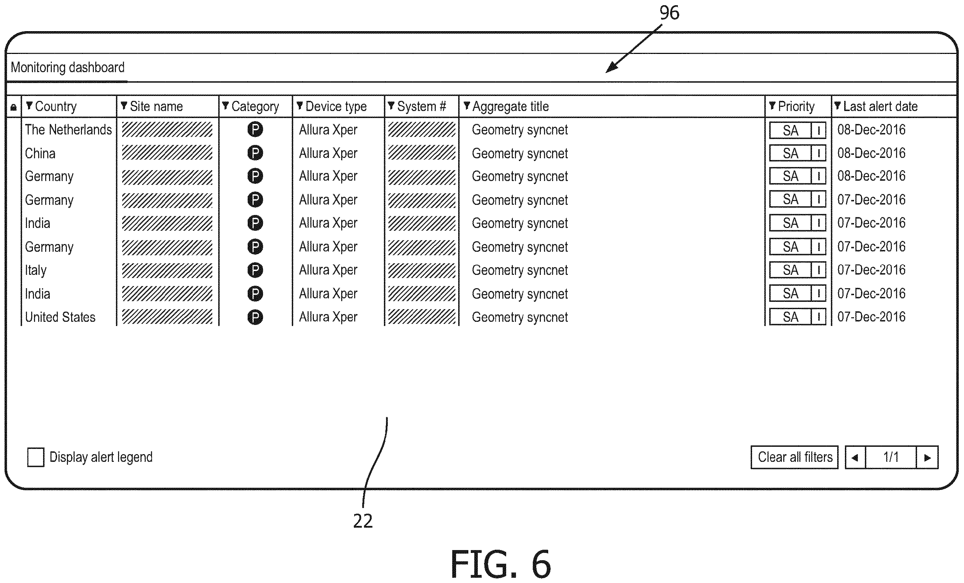

With returning reference to FIGURE 1 and further reference to FIGURE 6, an illustrative display (referred to as a "Monitoring Dashboard" **96**) is shown in FIGURE 6 of possible maintenance alerts generated by the predictive maintenance alerting device **40** of FIGURE 1 and suitably shown on the display **22** at the service center **12**. As seen in FIGURE 6, the left two columns identify the "Country" and "Site name" of the medical imaging device to which the maintenance alert pertains. (It is to be understood that the predictive maintenance alerting device **40** of FIGURE 1 may be employed to monitor and issue maintenance alerts for not just the single illustrative medical imaging device **10** shown in FIGURE 1, but rather for an entire fleet of medical imaging devices which may be deployed in different hospitals around the world, where in general the medical imaging devices may be of different types, e.g. CT, MRI, iGT, et cetera). In FIGURE 1 the content of the "Site name" column is redacted. Various other information may be provided about each alert as indicated in the "Category", "Device type", and "System #" columns. For example, the "Device type" column identifies the make and model of medical imaging device (where the illustrative "Allura XPer" refers to the Philips Allura Xper FD20/10 biplane mixed cardiovascular X-ray system as an illustrative example) and "System #" is a serial number or other unique identifier of the specific instance or installation. The "Aggregate Title" identifies the component group to which the maintenance alert pertains (e.g. the Geometry Syncnet component group in the illustrative example). The column headed "Priority" serves two purposes: the label on each icon identifies the priority, while the icon itself can be selected with a click of the mouse **26** (or by touching a touch screen, or by some other user input device) to bring up a pop-up window providing information on the details of the maintenance alert (e.g. the expected failure mode and expected remedial action to be required, and possibly other relevant information such as the time horizon over which the failure is expected to occur, taken from the statistical component lifetime models **72**). Finally, the last column headed "Last Alert Date" indicates the date of issuance of the maintenance alert.

The invention has been described with reference to the preferred embodiments. Modifications and alterations may occur to others upon reading and understanding the preceding detailed description. It is intended that the invention be construed as including all such modifications and alterations insofar as they come within the scope of the appended claims.

## Claims

1. A non-transitory storage medium storing instructions readable and executable by an electronic processor (42) to perform a predictive maintenance alerting method comprising:
receiving time stamped machine log data (30) and time stamped service log data (32) for a medical imaging device (10) via an electronic network (46);
deriving features from the received time stamped machine log data and time stamped service log data, including deriving at least one of (i) built failure mode features (62) each comprising a combination of two or more features that together represent a failure mode of a component and (ii) built failure resolution features (63) each comprising a combination of two or more features that together represent a failure of a component and a resolution of the failure of the component;
**characterized by** applying a set of models (64) of component groups to the derived features to generate maintenance alerts (44) wherein each component of the medical imaging device (10) is exclusively a member of a single component group; and transmitting the generated maintenance alerts to a service center (12) via the electronic network.

2. The non-transitory storage medium of claim 1 wherein at least one model of the set of models (64) of component groups comprises a heterogeneous model including a machine-learned analytical model (70) representing the component group with embedded statistical remaining useful lifetime models (72) of the components of the component group.

3. The non-transitory storage medium of any one of claims 1-2 wherein the deriving of features includes deriving built failure mode features (62) each comprising a combination of two or more features that together represent a failure mode of a component.

4. The non-transitory storage medium of any one of claims 1-3 wherein the deriving of features includes deriving built failure resolution features (63) each comprising a combination of two or more features that together represent a failure of a component and a resolution of the failure of the component.

5. The non-transitory storage medium of any one of claims 1-4 wherein the stored instructions are readable and executable by the electronic processor (42) to further perform a machine learning method operating on training data (82) comprising time stamped machine log data and time stamped service log data for at least one of the medical imaging device and one or more other medical imaging devices of a same type, the machine learning method comprising:
training the models (64) of the set of models of component groups using machine learning to optimize an objective (94) comprising maximizing true positive maintenance alerts for the component group subject to an upper limit on false positive maintenance alerts for the component group.

6. The non-transitory storage medium of any one of claims 1-5 wherein the stored instructions are readable and executable by the electronic processor (42) to further perform a machine learning method operating on training data (82) comprising time stamped machine log data and time stamped service log data for at least one of the medical imaging device and one or more other medical imaging devices of a same type, the machine learning method comprising:
extracting positive training data from the training data for training a model of the set of models of component groups wherein the positive training data comprise training data having time stamps pre-dating a failure of a component of component group;
extracting negative training data from the training data for training the model of the set of models of component groups wherein the negative training data comprise training data having time stamps post-dating a failure of a component of component group or training data from a medical imaging device that has never had a failure of the component of component group; and
applying machine learning to train the model using the positive training data and the negative training data.

7. The non-transitory storage medium of any one of claims 1-6 wherein the received time-stamped machine log data (30) includes medical imaging device usage log data and sensor data acquired by sensors of the medical imaging device.

8. A predictive maintenance alerting device comprising:
a server computer (42) operatively connected with an electronic network (46) to receive time stamped machine log data (30) and time stamped service log data (32) from a medical imaging device (10) via the electronic network and to transmit maintenance alerts (44) to a service center (12) via the electronic network; and
a non-transitory storage medium storing instructions readable and executable by the server computer to perform a predictive maintenance alerting method including:
deriving features from the received time stamped machine log data and time stamped service log data, including deriving at least one of (i) built failure mode features (62) each comprising a combination of two or more features that together represent a failure mode of a component and (ii) built failure resolution features (63) each comprising a combination of two or more features that together represent a failure of a component and a resolution of the failure of the component;; and
**characterized by** applying a set of models (64) of component groups to the derived features to generate the maintenance alerts (44) wherein each component of the medical imaging device (10) is exclusively a member of a single component group and wherein each model of the set of models of component groups comprises a heterogeneous model including a machine-learned analytical model (70) representing the component group with embedded statistical remaining useful lifetime models (72) of the components of the component group.

9. The predictive maintenance alerting device of claim 8 wherein the deriving of features includes deriving built failure mode features (62) each comprising a combination of two or more features that together represent a failure mode of a component.

10. The predictive maintenance alerting device of any one of claims 8-9 wherein the deriving of features includes deriving built failure resolution features (63) each comprising a combination of two or more features that together represent a failure of a component and a resolution of the failure of the component.

11. The predictive maintenance alerting device of any one of claims 8-10 wherein the stored instructions are readable and executable by the server computer (42) to further perform a machine learning method operating on training data (82) comprising time stamped machine log data and time stamped service log data for at least one of the medical imaging device and one or more other medical imaging devices of a same type, the machine learning method comprising:
training the models (64) of the set of models of component groups using machine learning to optimize an objective (94) comprising maximizing true positive maintenance alerts for the component group subject to an upper limit on false positive maintenance alerts for the component group.

12. The predictive maintenance alerting device of any one of claims 8-11 wherein the stored instructions are readable and executable by the server computer (42) to further perform a machine learning method operating on training data (82) comprising time stamped machine log data and time stamped service log data for at least one of the medical imaging device and one or more other medical imaging devices of a same type, the machine learning method comprising:
extracting positive training data from the training data for training a model of the set of models of component groups wherein the positive training data comprise training data having time stamps pre-dating a failure of a component of component group;
extracting negative training data from the training data for training the model of the set of models of component groups wherein the negative training data comprise training data having time stamps post-dating a failure of a component of component group or training data from a medical imaging device that has never had a failure of the component of component group; and
applying machine learning to train the model using the positive training data and the negative training data.

13. The predictive maintenance alerting device of any one of claims 8-12 wherein:
the set of models (64) of component groups model at least 10,000 components of the medical imaging device.

14. The predictive maintenance alerting device of any one of claims 8-13 wherein the predictive maintenance alerting method further includes at least one of:
maintaining an electronic service schedule (48) including scheduling a service call to remediate a generated maintenance alert; and
maintaining an electronic inventory (49) including ordering a part for the medical imaging device (10) that is expected to be needed to remediate a generated maintenance alert.

15. A predictive maintenance alerting method comprising:
receiving time stamped machine log data (30) and time stamped service log data (32) for a medical imaging device (10) at a server computer (42) via an electronic network (46);
deriving features from the received time stamped machine log data and time stamped service log data, including deriving at least one of (i) built failure mode features (62) each comprising a combination of two or more features that together represent a failure mode of a component and (ii) built failure resolution features (63) each comprising a combination of two or more features that together represent a failure of a component and a resolution of the failure of the component;
**characterized by** applying a set of models (64) of component groups to the derived features to generate maintenance alerts (44) wherein each component of the medical imaging device (10) is exclusively a member of a single component group; and
transmitting the generated maintenance alerts from the server computer to a service center (12) via the electronic network;
wherein the deriving and applying are performed by the electronic server.

## Patentansprüche

1. Nicht-transitorisches Speichermedium, das Anweisungen speichert, die von einem elektronischen Prozessor (42) lesbar und ausführbar sind, um ein Warnverfahren für die vorausschauende Wartung durchzuführen, umfassend:
Empfangen von zeitgestempelten Maschinenprotokolldaten (30) und zeitgestempelten Serviceprotokolldaten (32) für eine medizinische Bildgebungsvorrichtung (10) über ein elektronisches Netzwerk (46);
Ableiten von Funktionen aus den empfangenen zeitgestempelten Maschinenprotokolldaten und zeitgestempelten Serviceprotokolldaten, einschließlich Ableiten von mindestens einem von: (i) eingebauten Ausfallmodusfunktionen (62), die jeweils eine Kombination aus zwei oder mehr Funktionen umfassen, die zusammen einen Ausfallmodus einer Komponente darstellen, und (ii) eingebauten Ausfallbehebungsfunktionen (63), die jeweils eine Kombination aus zwei oder mehr Funktionen umfassen, die zusammen einen Ausfall einer Komponente und eine Behebung des Ausfalls der Komponente darstellen;
**gekennzeichnet durch** Anwenden eines Satzes von Modellen (64) von Komponentengruppen auf die abgeleiteten Funktionen, um Wartungswarnungen (44) zu erzeugen, wobei jede Komponente der medizinischen Bildgebungsvorrichtung (10) ausschließlich Mitglied einer einzigen Komponentengruppe ist; und Übertragen der erzeugten Wartungswarnungen über das elektronische Netzwerk an ein Servicecenter (12).

2. Nicht-transitorisches Speichermedium nach Anspruch 1, wobei mindestens ein Modell des Satzes von Modellen (64) von Komponentengruppen ein heterogenes Modell umfasst, das ein maschinengelerntes analytisches Modell (70) einschließt, das die Komponentengruppe mit eingebetteten statistischen Modellen der verbleibenden Nutzungsdauer (72) der Komponenten der Komponentengruppe darstellt.

3. Nicht-transitorisches Speichermedium nach einem der Ansprüche 1-2, wobei das Ableiten von Funktionen Ableiten eingebauter Ausfallmodusfunktionen (62) einschließt, die jeweils eine Kombination aus zwei oder mehr Funktionen umfassen, die zusammen einen Ausfallmodus einer Komponente darstellen.

4. Nicht-transitorisches Speichermedium nach einem der Ansprüche 1-3, wobei das Ableiten von Funktionen Ableiten eingebauter Ausfallbehebungsfunktionen (63) einschließt, die jeweils eine Kombination aus zwei oder mehr Funktionen umfassen, die zusammen einen Ausfall einer Komponente und eine Behebung des Ausfalls der Komponente darstellen.

5. Nicht-transitorisches Speichermedium nach einem der Ansprüche 1-4, wobei die gespeicherten Anweisungen vom elektronischen Prozessor (42) lesbar und ausführbar sind, um weiter ein maschinelles Lernverfahren durchzuführen, das auf Trainingsdaten (82) arbeitet, die zeitgestempelte Maschinenprotokolldaten und zeitgestempelte Serviceprotokolldaten für mindestens eine von der medizinischen Bildgebungsvorrichtung und einer oder mehreren anderen medizinischen Bildgebungsvorrichtungen eines selben Typs umfassen, wobei das maschinelle Lernverfahren Folgendes umfasst:
Trainieren der Modelle (64) des Satzes von Modellen von Komponentengruppen unter Verwendung von maschinellem Lernen, um ein Ziel (94) zu optimieren, das Maximieren echter positiver Wartungswarnungen für die Komponentengruppe vorbehaltlich einer Obergrenze für falsche positive Wartungswarnungen für die Komponentengruppe umfasst.

6. Nicht-transitorisches Speichermedium nach einem der Ansprüche 1-5, wobei die gespeicherten Anweisungen vom elektronischen Prozessor (42) lesbar und ausführbar sind, um weiter ein maschinelles Lernverfahren durchzuführen, das auf Trainingsdaten (82) arbeitet, die zeitgestempelte Maschinenprotokolldaten und zeitgestempelte Serviceprotokolldaten für mindestens eine von der medizinischen Bildgebungsvorrichtung und einer oder mehreren anderen medizinischen Bildgebungsvorrichtungen eines selben Typs umfassen, wobei das maschinelle Lernverfahren Folgendes umfasst:
Extrahieren positiver Trainingsdaten aus den Trainingsdaten zum Trainieren eines Modells des Satzes von Modellen von Komponentengruppen, wobei die positiven Trainingsdaten Trainingsdaten umfassen, die Zeitstempel aufweisen, die vor einem Ausfall einer Komponente der Komponentengruppe datiert sind;
Extrahieren negativer Trainingsdaten aus den Trainingsdaten zum Trainieren des Modells des Satzes von Modellen von Komponentengruppen, wobei die negativen Trainingsdaten Trainingsdaten umfassen, die Zeitstempel aufweisen, die nach einem Ausfall einer Komponente einer Komponentengruppe datiert sind, oder Trainingsdaten von einer medizinischen Bildgebungsvorrichtung, bei der nie ein Ausfall der Komponente einer Komponentengruppe aufgetreten ist; und
Anwenden von maschinellem Lernen, um das Modell mithilfe der positiven Trainingsdaten und der negativen Trainingsdaten zu trainieren.

7. Nicht-transitorisches Speichermedium nach einem der Ansprüche 1-6, wobei die empfangenen zeitgestempelten Maschinenprotokolldaten (30) Protokolldaten zur Verwendung der medizinischen Bildgebungsvorrichtung und von Sensoren der medizinischen Bildgebungsvorrichtung erfasste Sensordaten einschließen.

8. Warnvorrichtung für die vorausschauende Wartung, umfassend:
einen Servercomputer (42), der betriebsmäßig mit einem elektronischen Netzwerk (46) verbunden ist, um zeitgestempelte Maschinenprotokolldaten (30) und zeitgestempelte Serviceprotokolldaten (32) von einer medizinischen Bildgebungsvorrichtung (10) über das elektronische Netzwerk zu empfangen und Wartungswarnungen (44) über das elektronische Netzwerk an ein Servicecenter (12) zu übertragen; und
ein Nicht-transitorisches Speichermedium, das Anweisungen speichert, die vom Servercomputer lesbar und ausführbar sind zum Durchführen eines Warnverfahrens für die vorausschauende Wartung, einschließlich:
Ableiten von Funktionen aus den empfangenen zeitgestempelten Maschinenprotokolldaten und zeitgestempelten Serviceprotokolldaten, einschließlich Ableiten von mindestens einem von (i) eingebauten Ausfallmodusfunktionen (62), die jeweils eine Kombination aus zwei oder mehr Funktionen umfassen, die zusammen einen Ausfallmodus einer Komponente darstellen, und (ii) eingebauten Ausfallbehebungsfunktionen (63), die jeweils eine Kombination aus zwei oder mehr Funktionen umfassen, die zusammen einen Ausfall einer Komponente und eine Behebung des Ausfalls der Komponente darstellen; und
**gekennzeichnet durch** Anwenden eines Satzes von Modellen (64) von Komponentengruppen auf die abgeleiteten Funktionen, um die Wartungswarnungen (44) zu erzeugen, wobei jede Komponente der medizinischen Bildgebungsvorrichtung (10) ausschließlich Mitglied einer einzigen Komponentengruppe ist und wobei jedes Modell des Satzes von Modellen von Komponentengruppen ein heterogenes Modell umfasst, das ein maschinengelerntes analytisches Modell (70) einschließt, das die Komponentengruppe mit eingebetteten statistischen Modellen der verbleibenden Nutzungsdauer (72) der Komponenten der Komponentengruppe darstellt.

9. Warnvorrichtung für die vorausschauende Wartung nach Anspruch 8, wobei das Ableiten von Funktionen Ableiten eingebauter Ausfallmodusfunktionen (62) einschließt, die jeweils eine Kombination aus zwei oder mehr Funktionen umfassen, die zusammen einen Ausfallmodus einer Komponente darstellen.

10. Warnvorrichtung für die vorausschauende Wartung nach einem der Ansprüche 8-9, wobei Ableiten von Funktionen das Ableiten eingebauter Ausfallbehebungsfunktionen (63) einschließt, die jeweils eine Kombination aus zwei oder mehr Funktionen umfassen, die zusammen einen Ausfall einer Komponente und eine Behebung des Ausfalls der Komponente darstellen.

11. Warnvorrichtung für die vorausschauende Wartung nach einem der Ansprüche 8-10, wobei die gespeicherten Anweisungen vom Servercomputer (42) lesbar und ausführbar sind, um weiter ein maschinelles Lernverfahren durchzuführen, das auf Trainingsdaten (82) arbeitet, die zeitgestempelte Maschinenprotokolldaten und zeitgestempelte Serviceprotokolldaten für mindestens eine von der medizinischen Bildgebungsvorrichtung und einer oder mehreren anderen medizinischen Bildgebungsvorrichtungen desselben Typs umfassen, wobei das maschinelle Lernverfahren Folgendes umfasst:
Trainieren der Modelle (64) des Satzes von Modellen von Komponentengruppen unter Verwendung von maschinellem Lernen, um ein Ziel (94) zu optimieren, das Maximieren echter positiver Wartungswarnungen für die Komponentengruppe vorbehaltlich einer Obergrenze für falsche positive Wartungswarnungen für die Komponentengruppe umfasst.

12. Warnvorrichtung für die vorausschauende Wartung nach einem der Ansprüche 8-11, wobei die gespeicherten Anweisungen vom Servercomputer (42) lesbar und ausführbar sind, um weiter ein maschinelles Lernverfahren auszuführen, das auf Trainingsdaten (82) arbeitet, die zeitgestempelte Maschinenprotokolldaten und zeitgestempelte Serviceprotokolldaten für mindestens eine von der medizinischen Bildgebungsvorrichtung und einer oder mehreren anderen medizinischen Bildgebungsvorrichtungen desselben Typs umfassen, wobei das maschinelle Lernverfahren Folgendes umfasst:
Extrahieren positiver Trainingsdaten aus den Trainingsdaten zum Trainieren eines Modells des Satzes von Modellen von Komponentengruppen, wobei die positiven Trainingsdaten Trainingsdaten umfassen, die Zeitstempel aufweisen, die vor einem Ausfall einer Komponente der Komponentengruppe datiert sind;
Extrahieren negativer Trainingsdaten aus den Trainingsdaten zum Trainieren des Modells des Satzes von Modellen von Komponentengruppen, wobei die negativen Trainingsdaten Trainingsdaten umfassen, die Zeitstempel aufweisen, die nach einem Ausfall einer Komponente einer Komponentengruppe datiert sind, oder Trainingsdaten von einer medizinischen Bildgebungsvorrichtung, bei der nie ein Ausfall der Komponente einer Komponentengruppe aufgetreten ist; und
Anwenden von maschinellem Lernen, um das Modell mithilfe der positiven Trainingsdaten und der negativen Trainingsdaten zu trainieren.

13. Warnvorrichtung für die vorausschauende Wartung nach einem der Ansprüche 8-12, wobei:
der Satz von Modellen (64) von Komponentengruppen mindestens 10.000 Komponenten der medizinischen Bildgebungsvorrichtung modelliert.

14. Warnvorrichtung für die vorausschauende Wartung nach einem der Ansprüche 8-13, wobei das Warnverfahren für die vorausschauende Wartung weiter mindestens eines einschließt aus: Führen eines elektronischen Wartungsplans (48), einschließlich Planen eines Servicebesuchs zur Behebung eines erzeugten Wartungsalarms; und
Führen eines elektronischen Bestandsverzeichnisses (49) einschließlich Bestellen eines Teils für die medizinische Bildgebungsvorrichtung (10), das voraussichtlich benötigt wird, um einen erzeugten Wartungsalarm zu beheben.

15. Warnverfahren für die vorausschauende Wartung, umfassend:
Empfangen von zeitgestempelten Maschinenprotokolldaten (30) und zeitgestempelten Serviceprotokolldaten (32) für eine medizinische Bildgebungsvorrichtung (10) auf einem Servercomputer (42) über ein elektronisches Netzwerk (46);
Ableiten von Funktionen aus den empfangenen zeitgestempelten Maschinenprotokolldaten und zeitgestempelten Serviceprotokolldaten, einschließlich Ableiten von mindestens einem von: (i) eingebauten Ausfallmodusfunktionen (62), die jeweils eine Kombination aus zwei oder mehr Funktionen umfassen, die zusammen einen Ausfallmodus einer Komponente darstellen, und (ii) eingebauten Ausfallbehebungsfunktionen (63), die jeweils eine Kombination aus zwei oder mehr Funktionen umfassen, die zusammen einen Ausfall einer Komponente und eine Behebung des Ausfalls der Komponente darstellen;
**gekennzeichnet durch** Anwenden eines Satzes von Modellen (64) von Komponentengruppen auf die abgeleiteten Funktionen, um Wartungswarnungen (44) zu erzeugen, wobei jede Komponente der medizinischen Bildgebungsvorrichtung (10) ausschließlich Mitglied einer einzigen Komponentengruppe ist; und Übertragen der erzeugten Wartungswarnungen vom Servercomputer über das elektronische Netzwerk an ein Servicecenter (12);
wobei das Ableiten und Anwenden durch den elektronischen Server durchgeführt werden.

## Revendications

1. Support de stockage non transitoire stockant des instructions lisibles et exécutables par un processeur électronique (42) pour exécuter un procédé d'alerte de maintenance prédictive comprenant :
la réception de données de journal de machine horodatées (30) et des données de journal d'entretien horodatées (32) relatives à un dispositif d'imagerie médicale (10) par l'intermédiaire d'un réseau électronique (46) ;
la déduction de caractéristiques à partir des données de journal de machine horodatées et des données de journal d'entretien horodatées reçues, incluant la déduction d'au moins un élément parmi (i) des caractéristiques de mode de défaillance intégrées (62) comprenant chacune une combinaison de deux caractéristiques ou plus qui représentent ensemble un mode de défaillance d'un composant et (ii) des caractéristiques de résolution de défaillance intégrées (63) comprenant chacune une combinaison de deux caractéristiques ou plus qui représentent ensemble une défaillance d'un composant et une résolution de la défaillance du composant ;
**caractérisé par** l'application d'un ensemble de modèles (64) de groupes de composants aux caractéristiques déduites pour générer des alertes de maintenance (44), dans lequel chaque composant du dispositif d'imagerie médicale (10) est de manière exclusive un élément d'un groupe de composants unique ; et l'envoi des alertes de maintenance générées à un centre d'entretien (12) par l'intermédiaire du réseau électronique.

2. Support de stockage non transitoire selon la revendication 1, dans lequel au moins un modèle de l'ensemble de modèles (64) de groupes de composants comprend un modèle hétérogène incluant un modèle analytique appris automatiquement (70) représentant le groupe de composants avec des modèles statistiques de durée de vie utile restante intégrés (72) des composants du groupe de composants.

3. Support de stockage non transitoire selon l'une quelconque des revendications 1-2, dans lequel la déduction de caractéristiques inclut une déduction de caractéristiques de mode de défaillance intégrées (62) comprenant chacune une combinaison de deux caractéristiques ou plus qui représentent ensemble un mode de défaillance d'un composant.

4. Support de stockage non transitoire selon l'une quelconque des revendications 1-3, dans lequel la déduction de caractéristiques inclut une déduction de caractéristiques de résolution de défaillance intégrées (63) comprenant chacune une combinaison de deux caractéristiques ou plus qui représentent ensemble une défaillance d'un composant et une résolution de la défaillance du composant.

5. Support de stockage non transitoire selon l'une quelconque des revendications 1-4, dans lequel les instructions stockées sont lisibles et exécutables par le processeur électronique (42) pour exécuter en outre un procédé d'apprentissage automatique s'exécutant sur des données de formation (82) comprenant des données de journal de machine horodatées et des données de journal d'entretien horodatées relatives à au moins l'un du dispositif d'imagerie médicale et d'un ou plusieurs autres dispositifs d'imagerie médicale d'un même type, le procédé d'apprentissage automatique comprenant :
la formation des modèles (64) de l'ensemble de modèles de groupes de composants à l'aide d'un apprentissage automatique de façon à optimiser un objectif (94) comprenant une maximisation d'alertes de maintenance vraies positives relatives au groupe de composants sous réserve d'une limite supérieure associée à des alertes de maintenance fausses positives relatives au groupe de composants.

6. Support de stockage non transitoire selon l'une quelconque des revendications 1-5, dans lequel les instructions stockées sont lisibles et exécutables par le processeur électronique (42) pour exécuter en outre un procédé d'apprentissage automatique s'exécutant sur des données de formation (82) comprenant des données de journal de machine horodatées et des données de journal d'entretien horodatées relatives à au moins l'un du dispositif d'imagerie médicale et d'un ou plusieurs autres dispositifs d'imagerie médicale d'un même type, le procédé d'apprentissage automatique comprenant :
l'extraction de données de formation positives à partir des données de formation pour former un modèle de l'ensemble de modèles de groupes de composants, dans lequel les données de formation positives comprennent des données de formation présentant des horodatages antérieurs à une défaillance d'un composant d'un groupe de composants ;
l'extraction de données de formation négatives à partir des données de formation pour former le modèle de l'ensemble de modèles de groupes de composants, dans lequel les données de formation négatives comprennent des données de formation présentant des horodatages postérieurs à une défaillance d'un composant d'un groupe de composants ou des données de formation provenant d'un dispositif d'imagerie médicale qui n'a jamais connu de défaillance du composant du groupe de composants ; et
l'application d'un apprentissage automatique de façon à former le modèle en utilisant les données de formation positives et les données de formation négatives.

7. Support de stockage non transitoire selon l'une quelconque des revendications 1-6, dans lequel les données de journal de machine horodatées (30) reçues incluent des données de journal d'utilisation du dispositif d'imagerie médicale et des données de capteurs acquises par des capteurs du dispositif d'imagerie médicale.

8. Dispositif d'alerte de maintenance prédictive comprenant :
un ordinateur serveur (42) fonctionnellement connecté à un réseau électronique (46) pour recevoir des données de journal de machine horodatées (30) et des données de journal d'entretien horodatées (32) provenant d'un dispositif d'imagerie médicale (10) par l'intermédiaire du réseau électronique et pour envoyer des alertes de maintenance (44) à un centre d'entretien (12) par l'intermédiaire du réseau électronique ; et
un support de stockage non transitoire stockant des instructions lisibles et exécutables par l'ordinateur serveur pour exécuter un procédé d'alerte de maintenance prédictive incluant :
la déduction de caractéristiques à partir des données de journal de machine horodatées et des données de journal d'entretien horodatées reçues, incluant la déduction d'au moins un élément parmi (i) des caractéristiques de mode de défaillance intégrées (62) comprenant chacune une combinaison de deux caractéristiques ou plus qui représentent ensemble un mode de défaillance d'un composant et (ii) des caractéristiques de résolution de défaillance intégrées (63) comprenant chacune une combinaison de deux caractéristiques ou plus qui représentent ensemble une défaillance d'un composant et une résolution de la défaillance du composant ; et
**caractérisé par** l'application d'un ensemble de modèles (64) de groupes de composants aux caractéristiques déduites pour générer les alertes de maintenance (44), dans lequel chaque composant du dispositif d'imagerie médicale (10) est de manière exclusive un élément d'un groupe de composants unique et dans lequel chaque modèle de l'ensemble de modèles de groupes de composants comprend un modèle hétérogène incluant un modèle analytique appris automatiquement (70) représentant le groupe de composants avec des modèles statistiques de durée de vie utile restante intégrés (72) des composants du groupe de composants.

9. Dispositif d'alerte de maintenance prédictive selon la revendication 8, dans lequel la déduction de caractéristiques inclut une déduction de caractéristiques de mode de défaillance intégrées (62) comprenant chacune une combinaison de deux caractéristiques ou plus qui représentent ensemble un mode de défaillance d'un composant.

10. Dispositif d'alerte de maintenance prédictive selon l'une quelconque des revendications 8-9, dans lequel la déduction de caractéristiques inclut une déduction de caractéristiques de résolution de défaillance intégrées (63) comprenant chacune une combinaison de deux caractéristiques ou plus qui représentent ensemble une défaillance d'un composant et une résolution de la défaillance du composant.

11. Dispositif d'alerte de maintenance prédictive selon l'une quelconque des revendications 8-10, dans lequel les instructions stockées sont lisibles et exécutables par l'ordinateur serveur (42) pour exécuter en outre un procédé d'apprentissage automatique s'exécutant sur des données de formation (82) comprenant des données de journal de machine horodatées et des données de journal d'entretien horodatées relatives à au moins l'un du dispositif d'imagerie médicale et d'un ou plusieurs autres dispositifs d'imagerie médicale d'un même type, le procédé d'apprentissage automatique comprenant :
la formation des modèles (64) de l'ensemble de modèles de groupes de composants à l'aide d'un apprentissage automatique de façon à optimiser un objectif (94) comprenant une maximisation d'alertes de maintenance vraies positives relatives au groupe de composants sous réserve d'une limite supérieure associée à des alertes de maintenance fausses positives relatives au groupe de composants.

12. Dispositif d'alerte de maintenance prédictive selon l'une quelconque des revendications 8-11, dans lequel les instructions stockées sont lisibles et exécutables par l'ordinateur serveur (42) pour exécuter en outre un procédé d'apprentissage automatique s'exécutant sur des données de formation (82) comprenant des données de journal de machine horodatées et des données de journal d'entretien horodatées relatives à au moins l'un du dispositif d'imagerie médicale et d'un ou plusieurs autres dispositifs d'imagerie médicale d'un même type, le procédé d'apprentissage automatique comprenant :
l'extraction de données de formation positives à partir des données de formation pour former un modèle de l'ensemble de modèles de groupes de composants, dans lequel les données de formation positives comprennent des données de formation présentant des horodatages antérieurs à une défaillance d'un composant d'un groupe de composants ;
l'extraction de données de formation négatives à partir des données de formation pour former le modèle de l'ensemble de modèles de groupes de composants, dans lequel les données de formation négatives comprennent des données de formation présentant des horodatages postérieurs à une défaillance d'un composant d'un groupe de composants ou des données de formation provenant d'un dispositif d'imagerie médicale qui n'a jamais connu de défaillance du composant du groupe de composants ; et
l'application d'un apprentissage automatique de façon à former le modèle en utilisant les données de formation positives et les données de formation négatives.

13. Dispositif d'alerte de maintenance prédictive selon l'une quelconque des revendications 8-12, dans lequel :
l'ensemble des modèles (64) de groupes de composants modélise au moins 10 000 composants du dispositif d'imagerie médicale.

14. Dispositif d'alerte de maintenance prédictive selon l'une quelconque des revendications 8-13, dans lequel le procédé d'alerte de maintenance prédictive inclut en outre au moins l'une parmi : la gestion d'un calendrier d'entretien électronique (48), incluant la planification d'un appel d'entretien visant à corriger une alerte de maintenance générée ; et
la gestion d'un inventaire électronique (49), incluant la commande d'une pièce destinée au dispositif d'imagerie médicale (10) qui est censée être nécessaire pour corriger une alerte de maintenance générée.

15. Procédé d'alerte de maintenance prédictive comprenant :
la réception de données de journal de machine horodatées (30) et des données de journal d'entretien horodatées (32) relatives à un dispositif d'imagerie médicale (10) au niveau d'un ordinateur serveur (42) par l'intermédiaire d'un réseau électronique (46) ;
la déduction de caractéristiques à partir des données de journal de machine horodatées et des données de journal d'entretien horodatées reçues, incluant la déduction d'au moins un élément parmi (i) des caractéristiques de mode de défaillance intégrées (62) comprenant chacune une combinaison de deux caractéristiques ou plus qui représentent ensemble un mode de défaillance d'un composant et (ii) des caractéristiques de résolution de défaillance intégrées (63) comprenant chacune une combinaison de deux caractéristiques ou plus qui représentent ensemble une défaillance d'un composant et une résolution de la défaillance du composant ;
**caractérisé par** l'application d'un ensemble de modèles (64) de groupes de composants aux caractéristiques déduites pour générer des alertes de maintenance (44), dans lequel chaque composant du dispositif d'imagerie médicale (10) est de manière exclusive un élément d'un groupe de composants unique ; et l'envoi des alertes de maintenance générées depuis l'ordinateur serveur à un centre d'entretien (12) par l'intermédiaire du réseau électronique ;
dans lequel la déduction et l'application sont effectuées par le serveur électronique.
